# EUROPEAN PATENT APPLICATION

(11) **EP 4 368 241 A1**
(43) Date of publication of application: **15.05.2024**
(21) Application number: 23775951.9
(22) Date of filing: 08.06.2023
(51) Int. Cl.: A61N 1/32, A61N 1/06

(54) **PORTABLE BEAUTY INSTRUMENT**

(30) Priority: 30.09.2022 CN 202211217053; 28.10.2022 CN 202211339271; 28.10.2022 CN 202222874921 U; 28.10.2022 CN 202222875041 U; 28.10.2022 CN 202222874212 U; 01.11.2022 CN 202211361059; 01.11.2022 CN 202222919471 U; 01.11.2022 CN 202222919888 U; 01.11.2022 CN 202222907156 U
(71) Applicant: Shenzhen Ulike Smart Electronics Co., Ltd., Shenzhen, Guangdong 518000 (CN)
(72) Inventor: YANG, Jingjing, Shenzhen, Guangdong 518000 (CN)
(74) Representative: Torggler & Hofmann Patentanwälte - Innsbruck
(86) International application number: PCT/CN2023/099238
(87) International publication number: WO 2024/066468

(57) **Abstract**

This application discloses a portable beauty instrument, including a housing, including an end portion and a side portion that are used for contacting to-be-processed skin; and electrodes, including: a first electrode, where a part of the first electrode is located in the end portion of the housing, and a remaining part is located in the side portion of the housing; and third electrodes, disposed in the end portion or the side portion of the housing, where the third electrodes include group-C electrodes, and each of the group-C electrodes and an adjacent first electrode may form an electrode pair to generate a microcurrent. In this application, the first electrode is disposed in a connection region between the end portion and the side portion of the housing, and the first electrode may cooperate with the third electrode in the end portion or the side portion to form an electrode pair, so that the beauty instrument can discharge to a piece of local skin by using the first electrode, and also discharge to another piece of skin by using the third electrode. In addition, disposing the first electrode can further increase an electrode quantity of the beauty instrument, expand a range of skin to which a current of the beauty instrument can radiate, and enhance a cosmetic effect of the beauty instrument.

## Description

### TECHNICAL FIELD

Embodiments of this application relate to the field of electronic device technologies, and in particular, to a portable beauty instrument.

### BACKGROUND

With the advancement of technology and the progress of society, people are becoming increasingly concerned about skin care. While focusing on the effectiveness of skin care, there is also a growing emphasis on the overall experience of skin care. One of skin care manners is to process skin by using a portable beauty instrument with a radio frequency current or a microcurrent.

Beauty instruments on the market usually can only be used to process a large area of skin of a flat part, and cannot be used to locally process some edge skin with a relatively small area. Consequently, working modes are undiversified.

### SUMMARY

In view of the disadvantages in the conventional technology, this application provides a portable beauty instrument, to not only process a large area of skin of a flat part, but also locally process edge skin with a relatively small area, thereby enriching working modes of the portable beauty instrument.

To achieve the foregoing objective, this application uses the following technical solutions:

A portable beauty instrument is provided. The portable beauty instrument includes:
a housing, including an end portion and a side portion that are used for contacting to-be-processed skin; and
electrodes, including:
   a first electrode, where a part of the first electrode is located in the end portion of the housing, and a remaining part is located in the side portion of the housing; and
   third electrodes, disposed in the end portion or the side portion of the housing, where
   the third electrodes include group-C electrodes, and each of the group-C electrodes and an adjacent first electrode may form an electrode pair to generate a microcurrent.

In an implementation, the electrodes include two first electrodes, the first electrodes are arc-shaped electrode strips extending along an edge of the housing, and the two first electrodes are respectively located on two opposite sides of the end portion of the housing.

In an implementation, the group-C electrodes include two electrodes, the group-C electrodes are disposed in the end portion of the housing, and are located between the two first electrodes, and each of the first electrodes cooperates with an adjacent electrode in the group-C electrodes to form an electrode pair.

In an implementation, the third electrodes further include group-B electrodes that can independently work to generate a microcurrent or a radio frequency current, and electrodes in the group-B electrodes are arranged at intervals in a length direction of the first electrode.

In an implementation, the third electrodes are triangularly distributed as a whole, and the two electrodes in the group-C electrodes are respectively located on two sides of the group-B electrodes.

In an implementation, the electrodes further include:
second electrodes, disposed in the end portion of the housing, where the second electrodes cooperate to form an electrode pair that can discharge to the skin.

In an implementation, an outer side surface of the housing has a first region and a second region, an area of the first region is greater than an area of the second region, the second electrodes are disposed in the first region, and the third electrodes are disposed in the second region.

In an implementation, the second electrodes are located at one end of the third electrodes in an extension direction of the first electrode.

In an implementation, the second electrodes include a central electrode and several peripheral electrodes, and the several peripheral electrodes are disposed around the central electrode.

In an implementation, the central electrode is a ring-shaped electrode, the peripheral electrode is an arc segment-shaped electrode, and taking the central electrode as a center, the peripheral electrodes are distributed around the circumference of a specified circle at equal intervals.

In an implementation, the portable beauty instrument includes:
a processing circuit, located in the housing and connected to the electrodes; and
an adapter board, located in the housing and disposed near the end portion, where the adapter board has several connection terminals corresponding to the second electrodes and the third electrodes, the end portion has first through-holes corresponding to the second electrodes and the third electrodes, the second electrodes and the third electrodes are electrically connected to the several connection terminals through the first through-holes, and the several connection terminals are connected to the processing circuit.

In an implementation, the portable beauty instrument includes:
several first fasteners having conductivity;
where second through-holes are disposed at positions of the adapter board corresponding to the connection terminals, there are first fastening portions on sides of the second electrode and the third electrode facing the adapter board, and the first fasteners pass through the second through-holes from a side of the adapter board facing away from the second electrodes and the third electrodes, and are fastened to the first fastening portions.

In an implementation, all the peripheral electrodes in the second electrodes are connected through a connection line and/or a connection ring and/or a connection piece.

In an implementation, the portable beauty instrument includes:
a fastener, where
a mounting groove and a through-hole disposed at a groove bottom of the mounting groove are disposed in a connection region between the end portion and the side portion of the housing, there is a fastening portion on a side of the first electrode facing the housing, and the fastener passes through the through-hole from the inside of the housing, and is connected to the fastening portion; and
a sealing ring, sleeved on the fastening portion or the fastener and located between the first electrode and the groove bottom, to seal a gap between the fastening portion and an inner wall of the through-hole or seal a gap between the fastener and the inner wall of the through-hole.

In an implementation, the portable beauty instrument includes:
a circuit board, disposed between the adapter board and the end portion; and
several light therapy lamps, disposed on a side of the circuit board facing the end portion and connected to the processing circuit through the circuit board, where
the end portion has a transparent region corresponding to the light therapy lamp, and the transparent region is configured to shield an external line of sight when the light therapy lamp does not emit light and be capable of transmitting at least a part of light when the light therapy lamp emits light.

In an implementation, the portable beauty instrument includes:
a mounting bracket, disposed between the adapter board and the portion and having several positioning holes or positioning slots, where there is a light shielding region around the positioning holes or the positioning slots; and
a light guide member, disposed between the mounting bracket and the adapter board, where the light guide member includes a connecting bracket and several light guide columns, and the connecting bracket is connected between the several light guide columns, where
the positioning holes or the positioning slots, the light guide columns, and the light therapy lamps are disposed in a one-to-one correspondence.

In an implementation, the portable beauty instrument includes:
conductive members, connected to at least two of the electrodes and a processing circuit.

In an implementation, the portable beauty instrument further includes a gating circuit connected to the processing circuit, where the electrodes include second electrodes, where
the conductive members include a first conductive member and a second conductive member, the gating circuit is connected between the first conductive member and the second conductive member, and at least two of the second electrodes are connected to the first conductive member; and
at least two of the third electrodes are connected to the second conductive member, and the processing circuit drives the second electrodes, the third electrodes, or the second electrodes and the third electrodes by using the gating circuit.

In this application, the first electrode is disposed in a connection region between the end portion and the side portion of the housing, and the first electrode may cooperate with the third electrode in the end portion or the side portion to form an electrode pair, so that the beauty instrument can discharge to a piece of local skin by using the first electrode, and also discharge to another piece of skin by using the third electrode. In addition, disposing the first electrode can further increase an electrode quantity of the beauty instrument, expand a range of skin to which a current of the beauty instrument can radiate, and enhance a cosmetic effect of the beauty instrument.

### BRIEF DESCRIPTION OF DRAWINGS

FIG. 1 is a schematic diagram of a structure of a portable beauty instrument according to an embodiment of this application;
FIG. 2 is a side sectional view of a portable beauty instrument according to an embodiment of this application;
FIG. 3 is a schematic diagram of an assembly manner of a first electrode of a portable beauty instrument according to an embodiment of this application;
FIG. 4 is a schematic diagram of a structure of a middle frame of a portable beauty instrument according to an embodiment of this application;
FIG. 5 is a schematic diagram of an internal structure of a front housing of a portable beauty instrument according to an embodiment of this application;
FIG. 6 is a schematic diagram of electrode distribution of a portable beauty instrument according to an embodiment of this application;
FIG. 7 is a schematic diagram of an internal structure of a portable beauty instrument according to an embodiment of this application;
FIG. 8 is a schematic exploded view of a structure of an electrode of a portable beauty instrument according to an embodiment of this application;
FIG. 9 is a schematic exploded view of another structure of an electrode of a portable beauty instrument according to an embodiment of this application;
FIG. 10 is a schematic diagram of connecting a conductive member and a circuit in a portable beauty instrument according to an embodiment of this application;
FIG. 11 is a schematic diagram of an internal structure of a part of space of a portable beauty instrument according to an embodiment of this application;
FIG. 12 is a schematic diagram of a circuit structure of a portable beauty instrument according to an embodiment of this application;
FIG. 13 is a schematic diagram of an electrode connection manner of a portable beauty instrument according to an embodiment of this application;
FIG. 14 is a schematic diagram of a structure in which a first mounting plate of a portable beauty instrument faces a first side housing according to an embodiment of this application;
FIG. 15 is a schematic diagram of a structure in which a first mounting plate of a portable beauty instrument faces a second side housing according to an embodiment of this application;
FIG. 16 is a schematic diagram of a structure of an inner surface of a first side housing according to an embodiment of this application;
FIG. 17 is a schematic diagram of a structure of an inner surface of a second side housing according to an embodiment of this application;
FIG. 18 is a schematic diagram of a structure of a surface of a front housing according to an embodiment of this application; and
FIG. 19 is a schematic diagram of a three-dimensional structure of a portable beauty instrument according to an embodiment of this application.

Purpose implementation, functional features, and advantages of this application are further described with reference to the embodiments and the accompanying drawings.

### DESCRIPTION OF EMBODIMENTS

In this application, the terms "dispose", "provide", and "connect" shall be understood broadly, for example, may be a fastened connection, a detachable connection, or an integral structure, or may be a mechanical connection or an electrical connection, or may be a direct connection or an indirect connection using an intermediate medium, or may be a connection between two apparatuses, elements, or components. A person of ordinary skill in the art may understand specific meanings of the foregoing terms in this application based on specific conditions.

An orientation or positional relationship indicated by terms "center", "longitudinal", "traverse", "length", "width", "thickness", "up", "down", "front", "back", "left", "right", "vertical", "horizontal", "top", "bottom", "inside", "outside", "clockwise", "counter-clockwise", "axial", "radial", "circumferential", and the like is an orientation or positional relationship shown based on the accompanying drawings, is intended only to facilitate the description of this application and simplification of the description rather than indicating or implying that an apparatus or an element indicated must have a specific orientation, or be constructed and operated in a specific orientation, and therefore is not intended to be construed as a limitation to this application.

In addition, terms "first" and "second" are used only for description purposes, and cannot be understood as an indication or an implication of relative importance or an implicit indication of a quantity of indicated technical features. Therefore, features limited by "first" and "second" may explicitly or implicitly include at least one such feature. In the description of this application, "a plurality of" means at least two, for example, two or three, unless otherwise specifically limited.

In addition, some of the foregoing terms may be used to indicate an orientation or positional relationship, and may also be used to indicate another meaning. For example, in some cases, the term "up" may also be used to indicate an attachment relationship or a connection relationship. A person of ordinary skill in the art may understand specific meanings of these terms in this application based on specific conditions.

To make the objectives, technical solutions, and advantages of this application clearer, the following further describes this application in detail with reference to the accompanying drawings and the embodiments. It should be understood that the specific embodiments described herein are merely used to explain this application, but are not intended to limit this application.

When a user uses a beauty instrument to beautify skin, the user needs to fully attach electrodes of the beauty instrument to a contact surface of the skin, so that the electrodes can discharge to stimulate the skin. To improve a cosmetic effect of a current on the skin, the electrodes are usually disposed in an end portion of the beauty instrument. When the beauty instrument needs to be used to beautify skin in a local target region with a small area, it is difficult to control a quantity of electrodes that are in contact with and discharge to the skin and a contact area. Consequently, the electrodes may act on irrelevant skin around the target region. In addition, for some biological parts located at the edge, for example, the ala nasi, it is difficult for the electrodes of the beauty instrument to be attached to the biological parts. As a result, some biological parts cannot be stimulated by the current of the beauty instrument. Therefore, this application provides the following embodiments to expand an area of a biological part to which a current of a beauty instrument radiates.

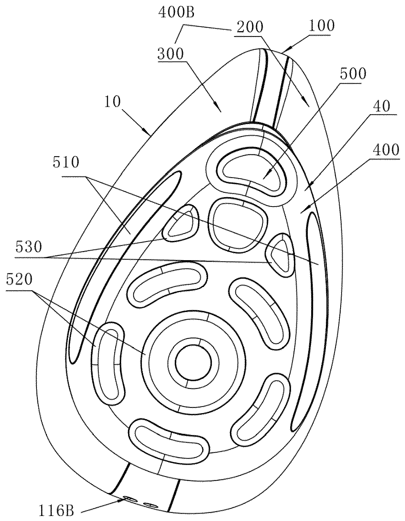

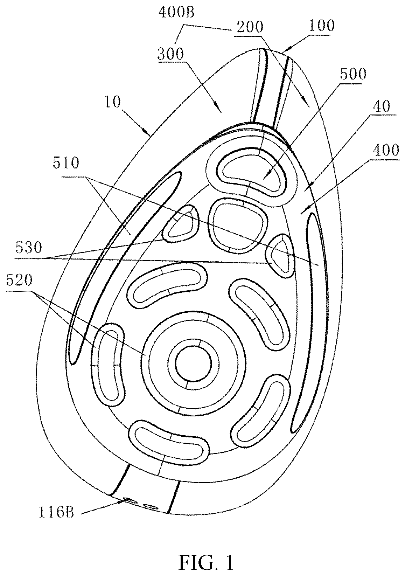

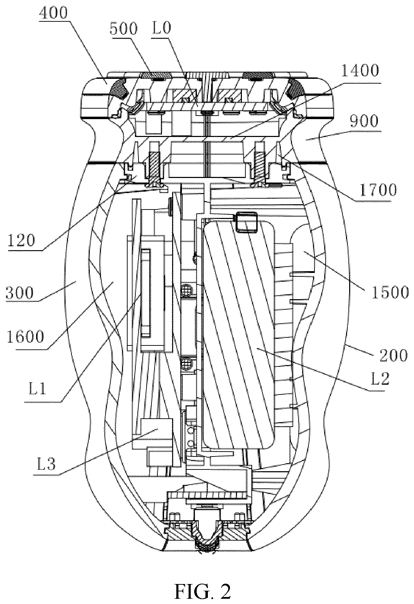

FIG. 1 is a schematic diagram of a structure of a portable beauty instrument according to an embodiment of this application. FIG. 2 is a side sectional view of a portable beauty instrument according to an embodiment of this application.

As shown in FIG. 1 and FIG. 2, the portable beauty instrument includes a housing 10, electrodes 500, and a processing circuit L1. The housing 10 includes an end portion and a side portion that are used for contacting to-be-processed skin. The electrodes 500 are disposed in regions of the end portion and the side portion of the housing 10, and are configured to discharge a current to stimulate the skin, thereby improving the skin. The processing circuit L1 is located in the housing 10, and is connected to the electrodes 500 to drive the electrodes 500 to discharge.

The electrodes 500 include a first electrode 510, second electrodes 520, and third electrodes 530. The first electrode 510 is disposed in a connection region between the end portion and the side portion of the housing 10, the second electrodes 520 are disposed in the end portion of the housing 10, and the third electrodes 530 are disposed in the end portion or the side portion of the housing 10. The third electrode 530 may generate a radio frequency current or a microcurrent, and cooperate with the first electrode 510 to form a first electrode pair, to generate a current to stimulate the skin, thereby improving the skin. In addition, there are at least two second electrodes 520, and the second electrodes 520 cooperate to form a second electrode pair. The first electrode pair and the second electrode pair may separately discharge to the skin or simultaneously discharge to the skin. A user may adjust a mode of the beauty instrument to control a quantity of electrode pairs that discharge, thereby controlling cosmetic strength of the beauty instrument.

The first electrode 510 and the second electrode 520 discharge currents to the skin, to expand a range of biological parts to which a current of the beauty instrument can radiate. For example, the user holds the side portion of the beauty instrument, and enables the end portion of the beauty instrument to come into contact with the skin, so that the second electrode 520 and the first electrode 510 can discharge to the skin by being attached to the skin. If the user wants to stimulate the ala nasi and surrounding biological parts, the user may tilt the beauty instrument, so that the first electrode 510 is attached to the ala nasi. For another example, if the user only wants to use the beauty instrument to process local skin with a relatively small area, the user may tilt the beauty instrument to attach the first electrode 510 to the skin to stimulate the local skin.

In an embodiment, there is an included angle of less than 120 degrees between the end portion and the side portion, and the connection region between the end portion and the side portion is a bent region between the end portion and the side portion. For example, there are two first electrodes 510, the two first electrodes 510 are respectively located on two opposite sides of the end portion, and each first electrode 510 extends along a bend line of the bent region, in other words, the first electrode 510 extends along the edge of the end portion, a part of each first electrode 510 is located in the end portion, a part is located on the bend line, and a remaining part is located in the side portion. Certainly, if the bend line is ignored, it may alternatively be considered that a part of each first electrode 510 is located in the end portion, and a remaining part is located in the side portion. For example, the included angle between the end portion and the side portion of the beauty instrument may be 110 degrees or 70 degrees. In this embodiment, the included angle between the end portion and the side portion is less than 120 degrees. When the beauty instrument is used to beautify some skin with a bending angle, for example, the ala nasi, the beauty instrument can be better attached to the skin of the part, to implement a tightening effect.

Certainly, in another embodiment, the first electrode 510 may be a dot electrode, and does not necessarily extend along the bend line of the bent region.

In a process of using the beauty instrument, the electrode 500 may generate a microcurrent, which acts on the skin and stimulates the skin, to lift and tighten the skin and effectively resist aging. The first electrodes 510 have two arc-shaped electrode strips that are located in the connection region between the end portion and the side portion of the housing 10. Because the first electrode 510 is an arc-shaped electrode strip, this design can make the electrode strip be better attached to facial contours, especially in a concave part of nasolabial folds on the face. Therefore, skin lifting and tightening effects are better.

In this embodiment, controlling the electrode 500 to generate a microcurrent can stimulate the skin and improve the skin, for example, implement at least one effect of quick skin lifting, strong anti-aging, skin elasticity enhancement, 3-minute V-face contouring, fading of nasolabial folds, tightening of the circumference of eyes, strengthening of a skin barrier, and achieving smooth and radiant skin.

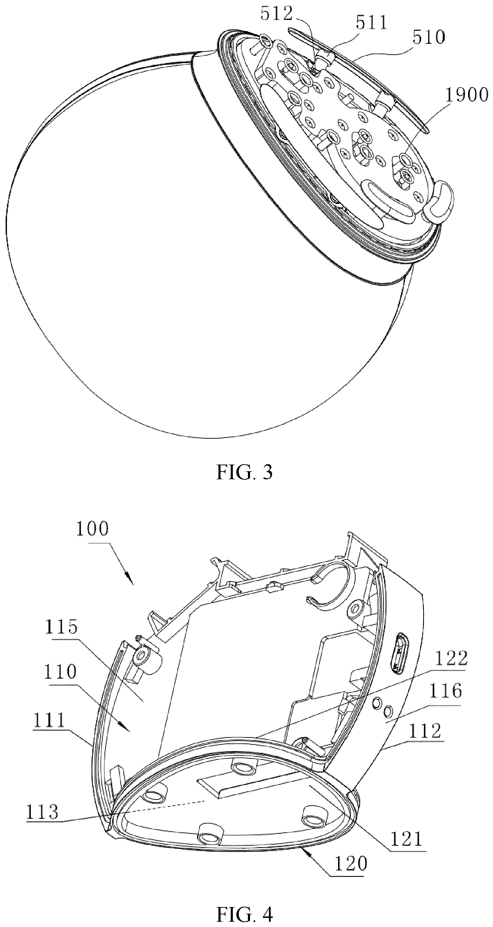

FIG. 3 is a schematic diagram of an assembly manner of a first electrode of a portable beauty instrument according to an embodiment of this application.

Referring to FIG. 3, the portable beauty instrument includes a fastener 512. The housing 10 is provided with a through-hole in the connection region, and there is a fastening portion 511 on a side of the first electrode 510 facing the housing 10. The fastener 512 is enabled to pass through the through-hole in the housing 10 from the inside of the housing 10 and connect to the fastening portion 511 of the first electrode 510, so that the first electrode 510 can be fastened to the housing 10.

The first electrode 510 is fastened in the following manner: A mounting position corresponding to the first electrode 510 in the housing 10 is found, and the fastening portion 511 of the first electrode 510 is enabled to pass through the through-hole reserved on the housing 10. Then, the fastening portion 511 that passes through the through-hole is found in the housing 10, and the fastener 512 is mounted on the fastening portion 511, so that the first electrode 510 is fastened to the housing 10. In this embodiment, the fastener 512 is disposed, and the fastener 512 is enabled to pass through the through-hole from the inside of the housing 10 and be fastened to the fastening portion 511 of the first electrode 510, so that the first electrode 510 is fastened to the housing 10, and the first electrode 510 can be stably fastened to a specified region without being shifted or loosened by an external force.

Optionally, a sealing ring 1900 is sleeved on the fastening portion 511 or the fastener 512, to seal a gap between the fastening portion 511 and an inner wall of the through-hole or seal a gap between the fastener 512 and the inner wall of the through-hole.

Because contact between the fastening portion 511 or the fastener 512 and the through-hole may be rigid contact, a deformation amount thereof may not be enough to block a gap between the fastening portion 511 or the fastener 512 and the through-hole that is caused by a machining error or the like. In this embodiment, the sealing ring 1900 is sleeved on the fastening portion 511 or the fastener 512, so that sealing and liquid penetration prevention performance of the beauty instrument can be enhanced. For example, the sealing ring 1900 can prevent a lotion, cosmetic gel, or the like from entering the housing 10 from the gap between the fastening portion 511 and the inner wall of the through-hole or the gap between the fastener 512 and the inner wall of the through-hole.

Optionally, the housing 10 is provided with a mounting groove in the connection region, a shape of the mounting groove is adapted to the first electrode 510, a groove depth of the mounting groove matches a height of the first electrode 510, the first electrode 510 is embedded in the mounting groove, the through-hole is disposed at a groove bottom of the mounting groove, and the sealing ring 1900 is located between the first electrode 510 and the groove bottom.

Because contact between the first electrode 510 and the groove bottom may be rigid contact, a deformation amount thereof may not be enough to block a gap between the first electrode 510 and the groove bottom that is caused by a machining error or the like. In addition, when the first electrode 510 is in direct contact with the skin, the beauty instrument may be used together with some liquids (for example, a lotion or skin care gel), and the liquids may enter the beauty instrument through the gap between the first electrode 510 and the groove bottom, affecting performance of the beauty instrument. Therefore, disposing the sealing ring 1900 between the first electrode 510 and the groove bottom can enhance sealing and liquid penetration prevention performance of a region of the beauty instrument for contacting the skin. For example, the sealing ring 1900 can prevent a lotion, cosmetic gel, or the like from entering the housing 10 from the gap between the fastening portion 511 and the inner wall of the through-hole or the gap between the fastener 512 and the inner wall of the through-hole.

Furthermore, in addition to preventing liquid penetration, the sealing ring 1900 can enable the first electrode 510 to be better assembled into the housing 10, so that the first electrode 510 does not move in a use process, thereby improving comfort of using the beauty instrument and facilitating use of the user.

FIG. 4 is a schematic diagram of a structure of a middle frame of a portable beauty instrument according to an embodiment of this application.

Referring to FIG. 1 and FIG. 4, in an embodiment, the portable beauty instrument includes a middle frame 100. The middle frame 100 has a first mounting plate 110 that extends in a first direction and a second mounting plate 120 that extends in a second direction. The first mounting plate 110 has a first side edge 111 and a second side edge 112 that are disposed opposite to each other and a third side edge 113 (blocked by the second mounting plate 120) disposed between the first side edge 111 and the second side edge 112. The second mounting plate 120 has a first surface 121 and a second surface 122 that are disposed opposite to each other. The second mounting plate 120 is connected to the third side edge 113 of the first mounting plate 110 through the second surface 122.

In this application, a basic framework of the beauty instrument is formed by using the middle frame, and therefore, the structure is simple and proper. When the user uses the beauty instrument to beautify the skin, the hand can move to a relatively small extent, which adapts to the ergonomics. Therefore, it is easy and effortless for the user to use the beauty instrument, so that use comfort is improved. In addition, in this application, a structure in which the electrodes and a side housing are mounted and an accommodation space for accommodating parts are formed by using the middle frame, and therefore, the structure is simple and proper.

Referring to FIG. 1, FIG. 2, and FIG. 4, the housing 10 includes a front housing assembly 40, a first side housing 200, and a second side housing 300. The front housing assembly 40 includes a front housing 400, and the electrode 500 is disposed on the front housing 400. A part of the electrode 500 is disposed in the front housing 400, and at least a part of the electrode 500 is exposed outside the front housing 400. The first side housing 200 and the second side housing 300 are respectively mounted on two opposite sides of the first mounting plate 110, and enclose with the first mounting plate 110 and the second mounting plate 120 respectively to form a first accommodation space 1500 and a second accommodation space 1600 separated by the first mounting plate 110. The processing circuit L1 is disposed in the second accommodation space 1600.

The front housing 400 directly faces the second surface 122 of the second mounting plate 120, and encloses with the second mounting plate 120 to form a third accommodation space 1700. The front housing 400 includes the foregoing end portion and side portion.

In this application, the housing 10 is divided into the front housing 400, the first side housing 200, and the second side housing 300. The front housing 400, the first side housing 200, and the second side housing 300 respectively form the third accommodation space 1700, the first accommodation space 1500, and the second accommodation space 1600 with the middle frame 100. A corresponding part is disposed in each accommodation space, a plurality of parts in the beauty instrument are partitioned, a structure is proper and compact, and mounting is convenient.

In this way, there are three accommodation spaces in the portable beauty instrument. The first mounting plate 110, the first side housing 200, and the second mounting plate 120 enclose to form the first accommodation space 1500, the first mounting plate 110, the second side housing 300, and the second mounting plate 120 enclose to form the second accommodation space 1600, the front housing 400, and the second mounting plate 120 enclose to form the third accommodation space 1700, and the first electrode 510 is located in the connection region between the end portion and the side portion of the front housing 400. In addition, the processing circuit L1, an energy storage circuit, a charging component, and the like are disposed in the first accommodation space 1500, the second accommodation space 1600, or the third accommodation space 1700.

In this embodiment, the first side housing 200 and the second side housing 300 are assembled together by using the middle frame 100, so that the beauty instrument can be assembled conveniently, and the first side housing 200 and the second side housing 300 have substantially same housing shapes, so that the housing of the beauty instrument is in a relatively regular shape, so that a user can better grab the beauty instrument.

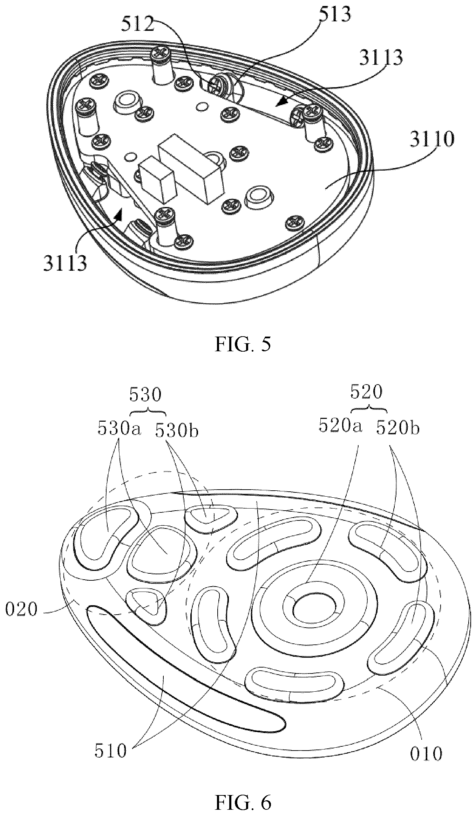

FIG. 5 is a schematic diagram of an internal structure of a front housing of a portable beauty instrument according to an embodiment of this application.

Referring to FIG. 2 and FIG. 5, in this embodiment, an inner surface of the connection region of the front housing 400 extends out of a fastening column 513 toward the third accommodation space 1700, the fastening column 513 extends in an inclined direction relative to the end portion of the front housing 400, a through-hole axially passes through the fastening column 513, and a fastener 512 is inserted into the through-hole. The fastener may be, for example, a screw. The electrode 500 may be locked in the front housing through cooperation of the fastener 512 and the fastening column 513, so that the electrode 500 can be stably fastened in a specified region without being offset and loosened by an external force.

Optionally, the processing circuit L1 includes a circuit board 3110, and an avoidance groove 3113 is formed at a position of the circuit board 3110 corresponding to the fastening column 513, so that the fastening column 513 is fastened to the circuit board 3110 by passing through the avoidance groove 3113, and the front housing 400 is fastened to the circuit board 3110 and is not offset and loosened due to an external force.

Optionally, in another embodiment of this application, the portable beauty instrument includes a spring pin assembly. A through-hole is disposed in the connection region of the housing 10, an aperture of one end of the through-hole adjacent to the inside of the housing 10 is greater than an aperture of one end thereof adj acent to the outside of the housing 10, and the first electrode 510 has a shape adapted to a shape of the through-hole, and is inserted into the through-hole through the inside of the housing 10 to be limited. The spring pin assembly is disposed between the first electrode 510 and the processing circuit L1, so as to electrically connect the first electrode 510 and the processing circuit L1.

In this way, the front housing 400 and the circuit board 3110 can be fastened together by using the spring pin assembly, and the first electrode 510 and the processing circuit L1 are electrically connected by using the spring pin assembly.

In this embodiment, the first electrode 510 is pluggable. Optionally, the user may unplug the first electrode 510, cover the end portion of the beauty instrument with a cleaning cloth/paper, and insert the first electrode 510 into a groove. In this way, the cleaning cloth/paper can be fastened on the end portion of the beauty instrument, and the user can clean the face by using the portable beauty instrument.

Optionally, in still another embodiment of this application, the portable beauty instrument includes a magnet. The housing 10 is provided with a groove in the connection region, the first electrode 510 is embedded in the groove, and the magnet is located in the first electrode 510 or the housing 10, so as to attract the first electrode 510 to the groove. The first electrode 510 and the housing 10 are connected and fastened by using a magnetic force.

The magnet is disposed in the first electrode 510 or the housing 10, so that the portable beauty instrument can add a cleaning function. The cleaning cloth/paper is sandwiched between the first electrode 510 and the front housing 400, and the user can use the portable beauty instrument to clean the skin. For example, a cloth/paper with a specific effect may also be sandwiched between the first electrode 510 and the front housing 400. The cloth/paper with a specific effect may be a facial mask, and the facial mask may be sandwiched between the first electrode 510 and the front housing 400, so as to accelerate a deep skin portion to absorb moisture of the facial mask to achieve a moisturizing and whitening effect.

Optionally, in another embodiment of this application, the first electrode 510 includes a ring electrode around the end portion of the housing 10. By disposing the ring electrode around the end portion of the housing 10, in a use process, the user can contact the skin with the first electrode 510 of the beauty instrument without intentionally adjusting an angle of the beauty instrument. In this way, in the use process, a hand movement range of the user may be smaller, conforming to ergonomics, making it convenient and effortless for the user, and enhancing use comfort. In addition, as a result of a decrease in hand movement and a decrease in shaking of the portable beauty instrument, the user feels better on the skin during cosmetic, and user experience is improved.

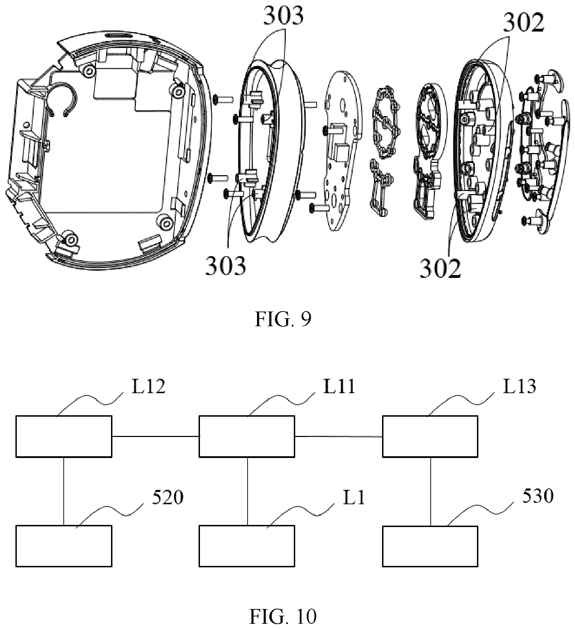

FIG. 6 is a schematic diagram of electrode distribution of a portable beauty instrument according to an embodiment of this application; and FIG. 10 is a schematic diagram of an internal structure of a portable beauty instrument according to an embodiment of this application.

Referring to FIG. 6 and FIG. 10, in an embodiment, the portable beauty instrument includes a housing 10, second electrodes 520, third electrodes 530, and a processing circuit L1. The housing 10 includes an end portion and a side portion that are used for contacting to-be-processed skin, an outer side surface of the end portion has a first region 010 and a second region 020, and an area of the first region 010 is greater than an area of the second region 020. The second electrodes 520 are disposed in the first region 010, and the third electrodes 530 are disposed in the second region 020. The processing circuit L1 is located in the housing 10, and is connected to the second electrodes 520 and the third electrodes 530.

For example, the first electrode 510 extends in a first direction, and the second electrodes 520 and the third electrodes 530 are sequentially arranged in the first direction.

The second electrodes 520 include a central electrode 520a and several peripheral electrodes 520b, and the several peripheral electrodes 520b are disposed around the central electrode 520a. The central electrode 520a is a ring-shaped electrode, the peripheral electrode 520b is an arc segment-shaped electrode, and taking the central electrode 520a as a center, the peripheral electrodes 520b are distributed around the circumference of a specified circle at equal intervals.

Because the size of the peripheral electrode 520b affects interaction between the electrodes, the peripheral electrodes 520b of different sizes generate different degrees of stimulation. For example, if the diameter of the peripheral electrode 520b is relatively small, a part of the peripheral electrode 520b that is in contact with the skin is too small, and the user cannot feel electrical stimulation. If the diameter of the peripheral electrode 520b is relatively large, a structure may also become large, a distance between the peripheral electrodes 520b may also become difficult to maintain, and a local stimulation effect may also become poor. Therefore, the size of the peripheral electrode 520b should be set properly according to an actual situation. In this way, even if output power of the peripheral electrode 520b is small, the user can easily feel it, which improves a cosmetic effect. In addition, even if power output of the peripheral electrode 520b is suppressed, the user can obtain strong stimulation, and therefore, a power saving effect can be implemented.

For example, in some embodiments, the diameter of the central electrode 520a may be from 8 mm to 12 mm, and the diameter of the peripheral electrode 520b may be from 2 mm to 6 mm.

The peripheral electrode 520b in this application is in a shape of an arc segment. Compared with a plane electrode in the past, a contact area between an arc segment-shaped electrode and skin is larger, and a current easily invades skin of the human body. Because a current flowing in an arc shape easily reaches the inside of the skin, a current of the peripheral electrode 520b can directly flow into the inside of the skin. That is, in this application, the peripheral electrode 520b in a shape of an arc segment can effectively apply the current to the inside of the skin.

In some embodiments, a quantity of peripheral electrodes 520b may be 4, 5, 6, or 8.Optionally, there are five peripheral electrodes 520b in this application.

In some embodiments, the third electrodes 530 may include group-B electrodes 530a and group-C electrodes 530b, where the group-B electrode 530a in the third electrodes 530 may generate a radio frequency current and a microcurrent as the second electrode 520. For example, the third electrodes 530 are triangularly distributed as a whole. As shown in FIG. 6, electrodes of the group-B electrodes 530a are arranged at intervals in a length direction of the first electrode 510, electrodes of the group-C electrodes 530b are respectively disposed on two sides of the group-B electrodes 530a, and the group-C electrodes 530b on two sides of the group-B electrodes 530a are respectively close to one arc-shaped first electrode 510. Two electrodes of the group-C electrodes 530b and one electrode of the group-B electrodes 530a enclose another electrode of the group-B electrodes 530a.

In some embodiments, the portable beauty instrument may further be provided with the first electrode 510 shown in FIG. 6. The first electrode 510 is an arc-shaped electrode strip, and may be better attached to a concave facial region, especially in a concave part of nasolabial folds on the face. Therefore, an effect of lifting and tightening the skin is better.

In an embodiment, the first electrodes 510 are separated by the third electrodes 530, and/or are separated by the second electrodes 520. As shown in FIG. 1, in a direction perpendicular to the first mounting plate 110, two first electrodes 510 are respectively located on two side edges of the housing 10, the second electrodes 520 and the third electrodes 530 are located between the two first electrodes 510, and in an extension direction of the first electrode 510, the second electrodes 520 are located at one end of the third electrodes 530.

For example, the group-C electrodes 530b may work with the arc-shaped electrode strip of the first electrode 510, and all group-C electrodes 530b may be positive electrodes or one thereof is a positive electrode. All arc-shaped electrode strips may be negative electrodes or one thereof is a negative electrode. That is, each of the group-C electrodes 530b may form a new electrode pair with an adjacent arc-shaped electrode strip, to generate a microcurrent.

For example, the second electrodes 520 occupy a relatively large range, and may act on a relatively flat large region, for example, a facial region, to improve working efficiency of the beauty instrument. The second electrodes 520 may generate a radio frequency current and a microcurrent, and send a soft electric wave to facial muscle, so as to stimulate vigor of the facial muscle, promote collagen regeneration, or stimulate the muscle, so as to tighten the skin. The third electrodes 530 occupy a relatively small range, and are triangularly distributed as a whole, and are more suitable for performing refinement work. For example, the group-B electrodes 530a in the third electrodes 530 work separately, and a microcurrent or a radio frequency current generated may act on a region such as the circumference of eyes. However, the first electrode 510 is an arc-shaped electrode strip. This design may enable the electrode strip to be better attached to the concave facial region, especially in a concave part of nasolabial folds on the face. In addition, the first electrode 510 may be combined with the group-C electrode 530b in the third electrodes 530 to generate a microcurrent. By generating a low-frequency current deep into the skin to stimulate muscle movement in a concave facial region (especially in a mandible region), the muscle is contracted. Therefore, the first electrode 510 and the group-C electrode 530b are mutually combined, and have a relatively good effect on skin lifting and tightening in the concave facial region.

With reference to the foregoing embodiments, a plurality of electrode groups may be disposed at the same time in the beauty instrument. Different electrode groups may work independently or together, and there may be a plurality of different combination manners between the plurality of electrode groups, so that a microcurrent or a radio frequency current is generated by the electrode and acts on the skin, thereby stimulating the skin, and achieving an effect of lifting and tightening the skin and effectively resisting aging.

In this embodiment, a plurality of electrode groups may be disposed, either working independently or in combination, to have different processing manners for different skin parts. Working modes and output energy corresponding to different combinations of electrode groups are also different. The electrode is controlled to generate a microcurrent and a radio frequency current to stimulate the skin, so that the user can select radio frequency cosmetic or microcurrent cosmetic according to a condition of the user, so as to improve the skin, for example, implement at least one cosmetic effect of quick skin lifting, strong anti-aging, skin elasticity enhancement, 3-minute V-face contouring, and fading of nasolabial folds. In addition, when the plurality of electrode groups work together, working efficiency of the beauty instrument can be further improved, thereby saving time for the user.

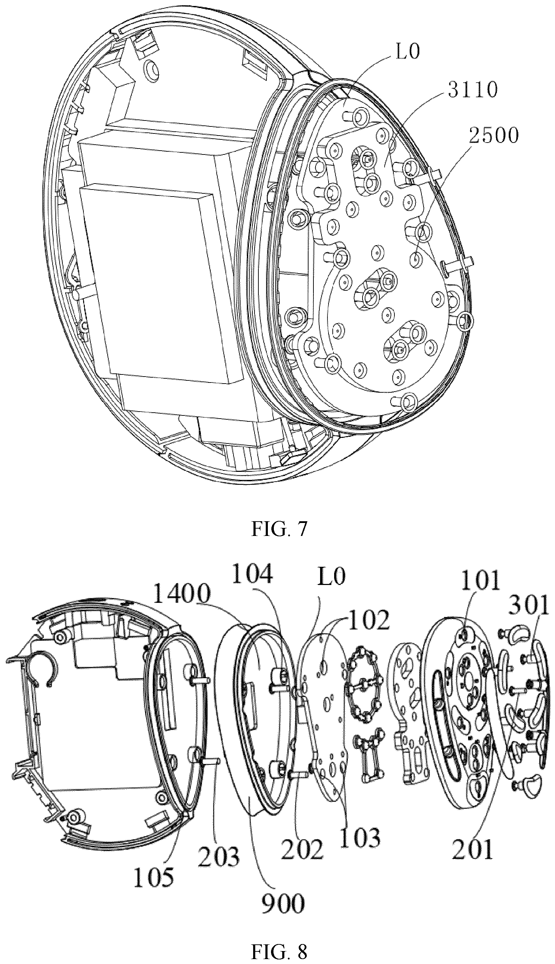

FIG. 7 is a schematic diagram of an internal structure of a portable beauty instrument according to an embodiment of this application; FIG. 8 is a schematic exploded view of a structure of an electrode of a portable beauty instrument according to an embodiment of this application; and FIG. 9 is a schematic exploded view of another structure of an electrode of a portable beauty instrument according to an embodiment of this application.

Referring to FIG. 1, FIG. 2, FIG. 4, and FIG. 6 to FIG. 9, in an embodiment, the portable beauty instrument includes an adapter board L0, where the adapter board L0 is located in the housing 10 and is disposed near the end portion, the adapter board L0 has several connection terminals corresponding to the second electrodes 520 and the third electrodes 530, the end portion has first through-holes 101 corresponding to the second electrodes 520 and the third electrodes 530, the second electrodes 520 and the third electrodes 530 are electrically connected to the several connection terminals by using the first through-holes 101, and the several connection terminals are connected to the processing circuit L 1.

In this way, the first through-holes 101 are located on the end portion of the housing 10, the second electrodes 520 and the third electrodes 530 are fastened to the end portion of the housing 10 by using the first through-holes 101, and are connected to the processing circuit L1 by using the several connection terminals on the adapter board L0.

For example, several first fasteners 201 have conductivity, and a second through-hole 102 is disposed at a position of a corresponding connection terminal of the adapter board L0. The second electrodes 520 and the third electrodes 530 have first fastening portions 301 on sides facing the adapter board L0. The first fastener 201 passes through the second through-hole 102 from a side of the adapter board L0 facing away from the second electrodes 520 and the third electrodes 530, and is fastened to the first fastening portion 301, so that the second electrodes 520 and the third electrodes 530 are electrically connected to the adapter board L0.

The second through-hole 102 is located on the adapter board L0, and the first fastener 201 passes through the second through-hole 102 from the side of the adapter board L0 facing away from the second electrodes 520 and the third electrodes 530. The first fastening portions 301 on the sides of the second electrodes 520 and the third electrodes 530 facing the adapter board L0 are connected to the first fastener 201, so that the second electrodes 520 and the third electrodes 530 are fastened to the adapter board L0, and the second electrodes 520 and the third electrodes 530 are electrically connected to the adapter board L0.

Optionally, in another embodiment of this application, at least two electrodes in the second electrodes 520 are electrically connected by using a connection line, and then the connection line is connected to the adapter board L0. In this way, the processing circuit L1 can reduce a connection operation between the electrode and the adapter board L0, and reduce processing difficulty. For example, all peripheral electrodes 520b of the second electrodes 520 are connected by using a connection line and/or a connection ring and/or a connection piece, and then connected to a connection terminal of the adapter board L0.

Optionally, in another embodiment of this application, at least two electrodes in the third electrodes 530 are electrically connected by using a connection line, and the connection line is connected to the processing circuit L1.

In another embodiment of this application, the portable beauty instrument includes several sealing rings (not shown in the figure). The first through-hole 101 is a stepped hole, each electrode of the second electrodes 520 is shaped to match the stepped hole, and is inserted into the stepped hole from the outside of the end portion and abuts against the stepped hole, and a sealing ring is sleeved on the second electrode 520 to seal a gap between the second electrode 520 and the first through-hole 101.

In some embodiments, a shape of the sealing ring may be specifically disposed according to a shape of the first through-hole 101. For example, if the shape of the first through-hole 101 is annular, the shape of the sealing ring may be correspondingly disposed as an O-type.

In some embodiments, the sealing ring may be of a rubber material. Because the rubber does not absorb water and is impervious to water, a sealing ring of the rubber material may block a relatively small gas or liquid channel that exists on an attached surface between the second electrode 520 and the first through-hole 101, so as to prevent water from entering the housing 10 through the gap between the second electrode 520 and the first through-hole 101, damaging a component such as a circuit board, and causing a failure of the beauty instrument to work normally.

Similarly, a sealing ring may be disposed on the third electrode 530, so as to prevent water from entering the housing from a gap between the third electrode 530 and the first through-hole 101.

The material of the sealing ring sleeved on the third electrode 530 may be an ultraviolet waterproof adhesive. The ultraviolet waterproof adhesive has a high solidification speed, high adhesive strength, good sealing performance, excellent water resistance and cold and hot resistance, and is safe and non-toxic. Therefore, water, gel, or other impurities can be effectively prevented from entering the housing from the gap between the third electrode 530 and the first through-hole 101, so that the third electrode 530 has good waterproof performance. In addition, the sealing ring sleeved on the third electrode 530 may also assist in fastening the third electrode 530, thereby improving stability of a connection between the third electrode 530 and the end portion.

It may be understood that the material of the sealing ring may alternatively be another material. A specific shape and material of the sealing ring are not strictly limited in this application.

With reference to FIG. 2, FIG. 6, and FIG. 7, in an embodiment, the portable beauty instrument includes a circuit board 3110 and several light therapy lamps 2500. The circuit board 3110 is disposed between the adapter board L0 and the end portion, and the several light therapy lamps 2500 are disposed on a side of the circuit board 3110 facing the end portion, and are connected to the processing circuit L1 by using the circuit board 3110.

The end portion of the housing 10 has a transparent region corresponding to the light therapy lamp 2500. The processing circuit L1 provides radio frequency output for the electrode and a drive signal for the light therapy lamp 2500. The transparent region of the end portion is configured to shield external line of sight when the light therapy lamp 2500 does not emit light. When the light therapy lamp 2500 emits light, at least a part of light may be transmitted, so that light can be prevented from being too dazzling to some extent, and light is weakened, so that the user can see softer light.

The light therapy lamp 2500 in this embodiment may emit light by using the transparent region at the end portion, and the processing circuit L1 provides a drive signal for the light therapy lamp 2500. In addition, the light therapy lamp 2500 may emit different light, and different light has different functions. Light can accelerate blood circulation of the skin, promote metabolism, improve cell activity, and help tissue repair. It can also inhibit oil secretion, inhibit skin inflammation, and regulate stratum corneum.

In an embodiment, as shown in FIG. 4, the first mounting plate 110 may include a first plate body 115 and two first side plates 116, the two first side plates 116 are respectively connected to opposite left and right sides of the first plate body 115, one end of each first side plate 116 in a width direction has a first side edge 111, and the other end has a second side edge 112.

In an embodiment, the first direction is perpendicular to the second direction. For example, the first direction is parallel to a central axis of the portable beauty instrument, and the second direction is perpendicular to the central axis of the portable beauty instrument.

In an embodiment, the first mounting plate 110 and the second mounting plate 120 each may be approximately a plate body, may be a frame body with a concave and convex structure, or may be a hollow structure.

Optionally, a third side edge 113 of the first mounting plate 110 is connected to a middle position of the second mounting plate 120.

With reference to FIG. 1, FIG. 2, FIG. 4, FIG. 8, and FIG. 9, in some embodiments, the portable beauty instrument further includes an annular housing 900 and several second fasteners 202. In addition to the front housing 400, the front housing assembly 40 further includes the annular housing 900, and the front housing 400, the annular housing 900, and the second mounting plate 120 enclose to form a third accommodation space 1700. The annular housing 900 is located between the front housing 400 and the second mounting plate 120 to connect the front housing 400 and the second mounting plate 120, and a partition plate 1400 is disposed in the annular housing 900. Several second fastening portions 302 are disposed on a side of the end portion of the housing 10 facing the adapter board L0, third through-holes 103 are disposed at positions of the adapter board L0 corresponding to the several second fastening portions 302, fourth through-holes 104 are disposed at positions of the partition plate 1400 corresponding to the third through-holes 103, and the second fastener 202 successively passes through the fourth through-hole 104 and the third through-hole 103 from the side of the partition plate 1400 facing away from the adapter board L0, and is fastened to the second fastening portion 302, so that the end portion of the housing 10, the adapter board L0, and the annular housing 900 are fastened.

In this way, the several second fasteners 202 pass through the third through-holes 103 on the adapter board L0 and the fourth through-holes 104 on the partition plate 1400, are connected in a one-to-one correspondence with the several second fastening portions 302 on the side of the end portion facing the adapter board L0, and fasten the end portion, the adapter board L0, and the annular housing 900.

Optionally, referring to FIG. 2, the portable beauty instrument includes a processing circuit L1, an energy storage circuit L2, a charging component L3, and the like. The processing circuit L 1, the energy storage circuit L2, and the charging component L3 are disposed in the first accommodation space 1500 and the second accommodation space 1600, the adapter board L0 is disposed in the third accommodation space 1700, and the adapter board L0 is connected to an electrode. The processing circuit L1 transmits a microcurrent and a radio frequency current to the electrode 500 by using the adapter board L0.

In this embodiment, the processing circuit L1 cooperates with the electrode to generate a microcurrent and a radio frequency current, so as to stimulate the skin, or irradiate the skin by using light emitted from the light therapy lamp or the like, thereby implementing effects such as quick skin lifting, strong anti-aging, skin elasticity enhancement, 3-minute V-face contouring, and fading of nasolabial folds.

In this embodiment, different components are placed in different accommodation spaces of the portable beauty instrument, which can prevent a temperature of the component from rising in a working process, thereby affecting a normal working situation of another component, and may also enable each component to be better fastened inside the portable beauty instrument.

In some embodiments, the portable beauty instrument further includes several third fasteners 203, where the partition plate 1400 is provided with several third fastening portions 303 on a side facing the second mounting plate 120, fifth through-holes 105 are disposed at positions of the second mounting plate 120 corresponding to the several second fastening portions 302, and the third fastener 203 passes through the fifth through-hole 105 on a side of the second mounting plate 120 facing away from the partition plate 1400, and is fastened to the third fastening portion 303, so that the front housing 400, the adapter board L0, the annular housing 900, and the middle frame 100 are fastened.

In this embodiment, the fifth through-hole 105 is disposed on the second mounting plate 120 of the middle frame 100, and after passing through the fifth through-hole 105, the third fastener 203 is connected in a one-to-one correspondence with the third fastening portion 303 disposed on the side of the partition plate 1400 facing the second mounting plate 120. Therefore, the front housing 400, the adapter board L0, the annular housing 900, and the middle frame 100 may be fastened by using the second fastening portion 302 and the third fastening portion 303.

In the foregoing embodiment, through cooperation of a through-hole, a fastener, and a fastening portion, mounting and removal of each component of the beauty instrument can be conveniently performed.

Optionally, in another embodiment of this application, the portable beauty instrument includes a sapphire (not shown in the figure) and a semiconductor refrigeration part (not shown in the figure), where the sapphire is disposed at the end portion and is exposed to an outer side of the end portion. The semiconductor refrigeration part is disposed on an inner side of the sapphire, and is thermally coupled to the sapphire and connected to the processing circuit L1. The processing circuit L1 is configured to drive the semiconductor refrigeration part to work, and cool or heat the sapphire, the second electrode 520, and/or the third electrode 530.

In some embodiments, a heat dissipation part may be further disposed in the portable beauty instrument. For example, the heat dissipation part may be thermally coupled to the semiconductor refrigeration part and/or the sapphire.

One side of the semiconductor refrigeration part is thermally coupled to a skin contact element, and the skin contact element may be the sapphire, the second electrode 520, and/or the third electrode 530. When the semiconductor refrigeration part generates heat, the skin contact element is heated. After the skin contact element contacts the skin, the skin feels comfortable and does not feel cold. When the semiconductor refrigeration part performs refrigeration, the skin contact element is cooled to lower the skin surface temperature when the skin is overheated.

Although the second electrode 520, the third electrode 530, and the first electrode 510 may generate a current, the muscle of the human body is stimulated by using the current, so that a passive movement of the muscle reaches a skin lifting and tightening effect, to further strengthen the cosmetic effect, some vibration components may be disposed on the portable beauty instrument.

For example, in an embodiment, a vibration component (not shown in the figure) may be further disposed in the third accommodation space 1700 of the portable beauty instrument, the vibration component is located on a side of the end portion facing inward, and is connected to the processing circuit L1.

Vibration of the vibration component can vibrate cells at the bottom layer of the skin, thus promoting skin care and enhancing cell activity. In addition, the vibration component has a relatively high vibration frequency, and includes a relatively large amount of energy, and can effectively massage cells of the skin, and break nutrients in a skin care product into small molecules, so that the skin can better absorb them. That is, the skin care product is better imported into the skin according to the vibration frequency, so that the skin is massaged, blood circulation is promoted, and a circulation function of the blood vessel is improved, so that the skin presents a healthier state.

In some embodiments, the vibration frequency of the vibration component can be adjusted. Therefore, the user can enable the vibration function of the beauty instrument, select a suitable vibration frequency to better import the skin care product into the skin, and massage the skin. For example, high-frequency vibration of a 8600 rpm rotation speed per minute may be set to help effective ingredients in the skin care product penetrate deeply into the skin to play a role.

In some embodiments, because the skin feeling of the skin is relatively poor at night, when the user uses the beauty instrument at night, the vibration function of the beauty instrument can be enabled, so that the beauty instrument vibrates in circular motions along the user's facial contours, helping effective ingredients in the skin care product penetrate deeply into the skin to play a role.

Optionally, in another embodiment of this application, the portable beauty instrument, the second electrode 520, and/or the third electrode 530 protrude from or are flush with the outer side of the end portion of the housing 10.

For ease of electrode mounting and correction, generally, the second electrode 520 and the third electrode 530 may simultaneously protrude from the outer side of the end portion, as shown in FIG. 6 of this application.

For example, a height by which the second electrode 520 and/or the third electrode 530 protrude from the outer side of the end portion of the housing 10 may be 2 mm to 3 mm.

In another embodiment, the second electrode 520 may be flush with the outer side of the end portion of the housing 10, the third electrode 530 protrudes from the outer side of the end portion of the housing 10, or the second electrode 520 protrudes from the outer side of the end portion of the housing 10, and the third electrode 530 is flush with the outer side of the end portion of the housing 10.

In another embodiment, alternatively, both the second electrode 520 and the third electrode 530 may be flush with the outer side of the end portion of the housing 10.

A specific disposing relationship between the position of the end portion and each of the second electrode 520 and the third electrode 530 is not limited in this application.

FIG. 10 is a schematic diagram of connecting a conductive member and a circuit in a portable beauty instrument according to an embodiment of this application.

Referring to FIG. 10, in some embodiments, the portable beauty instrument may further include a gating circuit L11 and conductive members. The conductive members include a first conductive member L12 and a second conductive member L13. The gating circuit L11 is connected between the first conductive member L12 and the second conductive member L13. At least two electrodes of the second electrodes 520 are connected to the first conductive member L12. At least two electrodes of the third electrodes 530 are connected to the second conductive member L13. The processing circuit L1 is connected to the gating circuit L 11, and drives the second electrodes 520 or the third electrodes 530, or drives the second electrodes 520 and the third electrodes 530 by using the gating circuit L11.

In some embodiments, the conductive member may be a wire, a spring pin, a conductive plate, or a circuit board.

Therefore, connection points between the electrode and the adapter board L0 can be reduced by using functions of the gating circuit L11 and the conductive members, and when the processing circuit L1 is connected to the gating circuit L11, the gating circuit L11 may further be used to independently drive the second electrodes 520 or the third electrodes 530 to work, or drive the second electrodes 520 and the third electrodes 530 to work. Therefore, different electrode groups may work independently or together. Different processing manners are used for different skin parts, and corresponding working modes and output energy are also different. If a plurality of electrode groups work together, working efficiency of the beauty instrument can be improved, thereby saving time for the user.

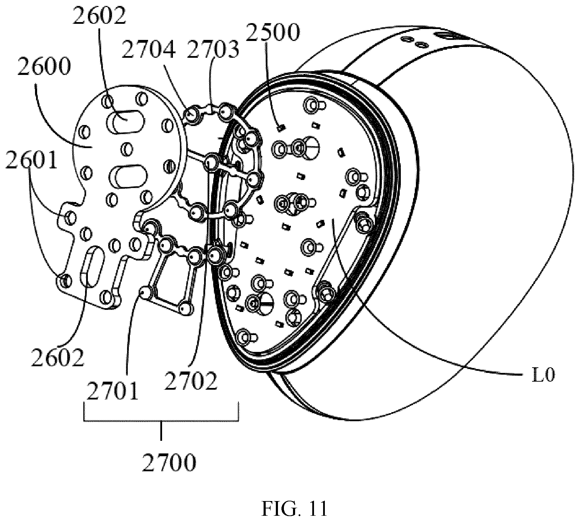

FIG. 11 is a schematic diagram of an internal structure of a part of space of an embodiment of a portable beauty instrument according to an embodiment of this application.

Referring to FIG. 11, in some embodiments, the portable beauty instrument may further include a mounting bracket 2600 and a light guide member 2700 that are disposed in the third accommodation space 1700. The mounting bracket 2600 is disposed between the adapter board L0 and the end portion of the housing 10, has several positioning holes or positioning slots 2601, and has a light shielding region around the positioning holes or positioning slots 2601. The light guide member 2700 is disposed between the mounting bracket 2600 and the adapter board L0, where the light guide member 2700 includes a first light guide portion 2701 and a second light guide portion 2702, each light guide portion has a connecting bracket 2703 and several light guide columns 2704, and the connecting bracket 2703 is connected between the several light guide columns 2704. Several light therapy lamps 2500 are disposed on the adapter board L0, and the light therapy lamp 2500 is connected to the processing circuit L1 by using the adapter board L0. The end portion has a transparent region corresponding to the light therapy lamp 2500, and the positioning holes and/or the positioning slots 2601, the light guide columns 2704, and the light therapy lamps 2500 are disposed in a one-to-one correspondence. The processing circuit L1 provides radio frequency output for the electrode and provides a drive signal for the light therapy lamp.

Because the positioning holes and/or positioning slots 2601 on the mounting bracket 2600 are disposed in a one-to-one correspondence with the light guide columns 2704 on the light guide member 2700 and the light therapy lamps 2500 on the adapter board L0, when three fastening holes 2602 on the mounting bracket 2600 are fastened to the electrodes, the light guide member 2700 disposed between the mounting bracket 2600 and the adapter board L0 is press-fit to the adapter board L0, so that the positioning holes or positioning slots 2601 on the mounting bracket 2600 are corresponding to and press-fit to the light guide columns 2704 on the light guide member 2700 and the light therapy lamps 2500 on the adapter board L0. The top of the light guide column 2704 is inserted into a corresponding positioning hole or positioning slot, so that when the processing circuit L1 provides radio frequency output for the electrode and provides a drive signal for the light therapy lamp 2500, the light guide member 2700 can aggregate light emitted by the light therapy lamp 2500. In addition, because there is a light shielding region around the positioning hole and/or positioning slot 2601 on the mounting bracket 2600, light shielding may be further performed.

In some embodiments, a material of the mounting bracket 2600 may be a silicone material.

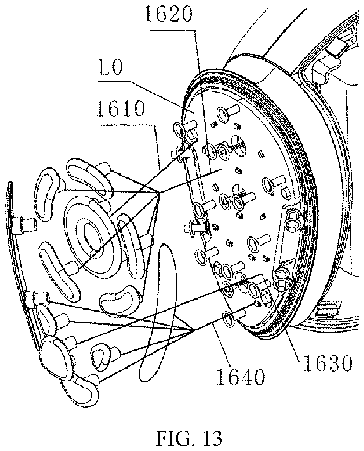

In an embodiment, the portable beauty instrument includes a front housing 400, several electrodes 500, a middle frame 100, a rear housing 400B, an annular housing 900, a processing circuit L1, and conductive members 1610 (refer to FIG. 13). The rear housing 400B (refer to FIG. 1) is used for holding, the front housing 400 is used for contacting the skin, and the several electrodes 500 are disposed in the front housing 400.

The rear housing 400B encloses at least a part of the first mounting plate 110, and forms an accommodation space with the first mounting plate 110. The processing circuit L1 is located in the accommodation space. The conductive members 1610 are connected to at least two electrodes of the electrodes 500 and the processing circuit L1.

Optionally, there are two accommodation spaces in the portable beauty instrument. The first mounting plate 110 surrounded by the rear housing 400B encloses to form a second accommodation space 1600, the front housing 400 and the second mounting plate 120 enclose to form a third accommodation space 1700, and the electrodes 500 are disposed on the front housing 400.

Optionally, the portable beauty instrument includes the processing circuit L1, an energy storage circuit L2, a charging component L3, and the like. The processing circuit L1, the energy storage circuit L2, and the charging component L3 are disposed in the second accommodation space 1600, the electrodes 500, the adapter board L0, and the like are disposed in the third accommodation space 1700, and the adapter board L0 is connected to the electrodes 500. The processing circuit L1 transmits a microcurrent and a radio frequency current to the electrodes 500 by using the adapter board L0.

Optionally, the several electrodes 500 include a positive electrode and a negative electrode, a quantity of the negative electrodes is at least two, all the negative electrodes are connected to the conductive members, and the positive electrode is connected to the processing circuit L 1.

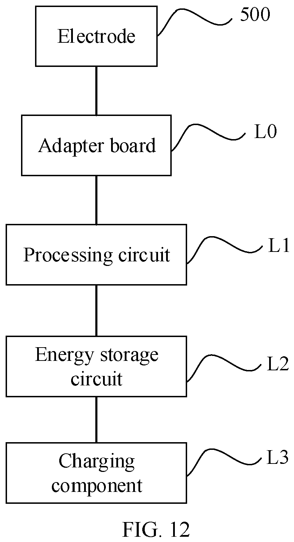

Referring to FIG. 10 to FIG. 13, the portable beauty instrument includes a gating circuit L11. The electrodes 500 include second electrodes 520 and third electrodes 530. The conductive members include a first conductive member L12 and a second conductive member L13. The gating circuit L11 is connected between the first conductive member L12 and the second conductive member L13. At least two electrodes 500 of the second electrodes 520 are connected to the first conductive member L12. At least two electrodes 500 of the third electrodes 530 are connected to the second conductive member L13. The processing circuit L1 is connected to the gating circuit L11, and drives the second electrodes 520 or the third electrodes 530, or drives the second electrodes 520 and the third electrodes 530 by using the gating circuit L11.

In this embodiment, the conductive member may be a third conductive member, such as a wire, configured to connect the electrode 500 to the adapter board L0. The electrode connected to the third conductive member 1610 is a positive electrode of the second electrodes 520, and is connected to the adapter board L0. The electrode connected to the third conductive member 1620 is a negative electrode of the second electrodes 520, and is connected to the adapter board L0. The electrode connected to the third conductive member 1630 is a positive electrode of the third electrodes 530, and is connected to the adapter board L0. The electrode connected to the third conductive member 1640 is a negative electrode of the third electrodes 530, and is connected to the adapter board L0. Electrodes 500 with a same attribute can be connected together by using a conductive member, thereby reducing connection points between the electrode 500 and the adapter board L0. In addition, when the processing circuit L1 is connected to the gating circuit L11, the gating circuit L11 may further be used to independently drive the second electrodes 520 or the third electrodes 530 to work, or drive the second electrodes 520 and the third electrodes 530 to work. For example, the processing circuit L1 controls the gating circuit L11 to drive the third conductive member 1610 and the third conductive member 1620, so that the second electrodes 520 work. Alternatively, the processing circuit L1 controls the gating circuit L11 to drive the third conductive member 1630 and the third conductive member 1640, so that the third electrodes 530 work. Alternatively, the processing circuit L1 controls the gating circuit L11 to drive the third conductive member 1610, the third conductive member 1620, the third conductive member 1630, and the third conductive member 1640, so that the second electrodes 520 and the third electrodes 530 work simultaneously. Therefore, different electrode groups may work independently or together. Different processing manners are used for different skin parts, and corresponding working modes and output energy are also different. If a plurality of electrode groups work together, working efficiency of the beauty instrument can be improved, thereby saving time for the user.

In this embodiment, when the front housing 400 is in contact with the skin, a positive electrode and a negative electrode are connected, and a microcurrent and a radio frequency current are generated by the electrodes, so that the skin can be stimulated, and the skin can be improved, for example, at least one cosmetic effect of quick skin lifting, strong anti-aging, skin elasticity enhancement, 3-minute V-face contouring, fading of nasolabial folds, tightening of the circumference of eyes, strengthening of a skin barrier, and achieving smooth and radiant skin is implemented.

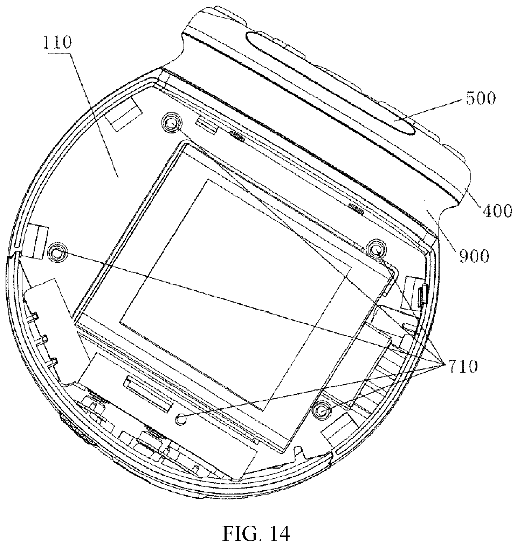

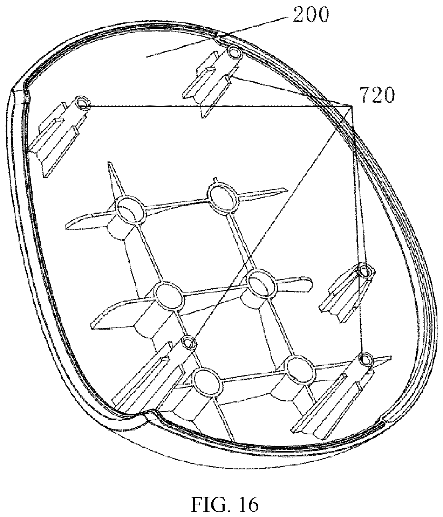

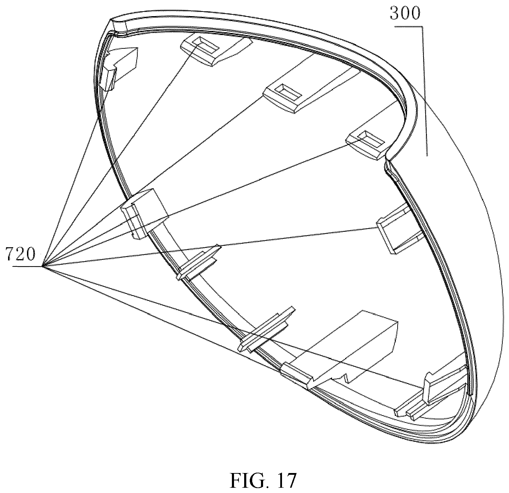

Referring to FIG. 1 and FIG. 14 to FIG. 17, in an embodiment, in the portable beauty instrument, the rear housing 400B includes a first side housing 200 and a second side housing 300, and the first side housing 200 and the second side housing 300 are respectively mounted on two opposite sides of the first mounting plate 110. A first clamping portion 710 is disposed on each of the two opposite sides of the first mounting plate 110, and a second clamping portion 720 is disposed on each of inner sides of the first side housing 200 and the second side housing 300 facing the first mounting plate 110. The first side housing 200 and the second side housing 300 are clamped in the first clamping portion 710 by using the second clamping portion 720.

Clamping manners of the first side housing 200 and the second side housing 300 are different, the first side housing 200 is clamped in the first mounting plate 110 in a circular embedding manner, and the second side housing 300 is clamped in the first mounting plate 110 in a square buckling manner.

Therefore, in this embodiment, the middle frame 100 is used as a backbone, the first side housing 200, the second side housing 300, and the front housing 400 are integrated at different positions of the middle frame 100, and the first side housing 200, the second side housing 300, and the front housing 400 together with the exposed first side edge 111 and second side edge 112 of the first mounting plate 110 and the exposed side edge of the second mounting plate 120 constitute the housing of the portable beauty instrument.

Referring to FIG. 2, the first side housing 200 and the second side housing 300 are collectively referred to as a rear housing 400B, and the two side housings have two opposite recesses, that is, each of the first side housing 200 and the second side housing 300 has one recess. In addition, the front housing 400 has a ring groove at a position adjacent to the rear housing 400B, where the recess and the ring groove conform to ergonomics, facilitating the user's grip on the beauty instrument during use, avoiding release from the hand.

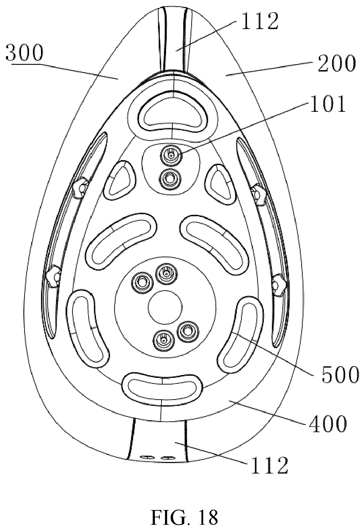

With reference to FIG. 18, the portable beauty instrument includes an adapter board L0, the adapter board L0 is located between the front housing 400 and the annular housing 900, the adapter board L0 has a connection terminal, the front housing 400 has first through-holes 101 corresponding to several electrodes 500, at least two electrodes 500 are electrically connected to the conductive member by using the first through-holes 101, and the conductive member is connected to the processing circuit L1 by using the connection terminal on the adapter board L0.

In this way, the processing circuit L1 outputs a radio frequency current or a microcurrent by using the connection terminal of the adapter board L0, and then transmits the radio frequency current or the microcurrent to the electrode 500 by using the conductive member.

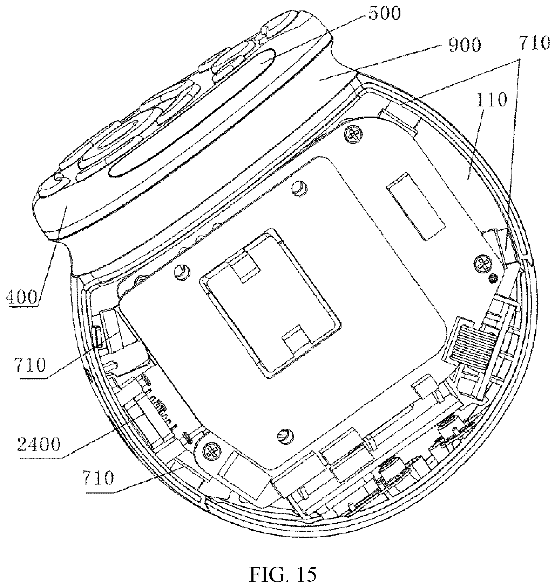

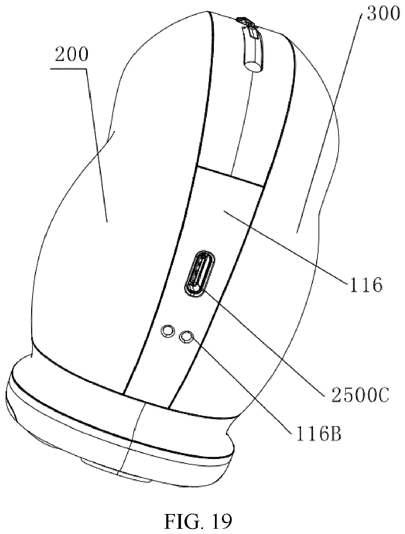

Referring to FIG. 15 and FIG. 19, in an embodiment, a charging groove 2400 is disposed on the first side plate 116, an avoidance groove 2500C is disposed at a position of the first plate body 115 adjacent to the charging groove 2400, the avoidance groove 2500C is configured to mount a charging plate, and the charging plate is connected to the charging groove 2400.

In an embodiment, the first side plate 116 is provided with a charging electrode 116B connected to the charging plate, and the charging electrode 116B is exposed to an outer surface of the first side plate 116.

In this way, the portable beauty instrument has two charging interfaces on the first side plate 116, and may provide two charging manners. The first manner is: connecting the charging groove 2400 to a data cable, where a current of the data cable enters the charging plate from the charging groove 2400, so as to implement charging of the charging plate. In the second manner, when the beauty instrument is correctly placed on a charging base of the beauty instrument, a current of the charging base is transmitted to the charging plate by using the charging electrode 116B, so as to implement charging of the charging plate.

Optionally, in another embodiment of this application, a wireless charging coil connected to the charging plate is disposed on the first side plate 116 of the portable beauty instrument. Only the wireless charging coil of the beauty instrument needs to be correctly corresponding to a wireless charging coil of the charging plate. When a varying current with a fixed frequency passes through the charging plate, a magnetic field is generated between the beauty instrument and the charging plate, and the wireless charging coil of the beauty instrument generates an induced current, to implement charging of the charging plate.

Optionally, in another embodiment of this application, the electrode 500 in the portable beauty instrument is integrally formed with the front housing 400, and the electrode 500 is exposed to the outer surface of the front housing 400.

When the electrode 500 is integrally formed with the front housing 400, water can be prevented from entering the inside of the front housing 400 from a mounting gap between the electrode 500 and the front housing 400.

The foregoing descriptions are merely specific implementations of this application. It should be noted that a person of ordinary skill in the art may make some improvements and ornaments without departing from the principle of this application. These improvements and ornaments shall also be considered as being within the protection scope of this application.

## Claims

1. A portable beauty instrument, comprising:
a housing, comprising an end portion and a side portion that are used for contacting to-be-processed skin; and
electrodes, comprising:
a first electrode, wherein a part of the first electrode is located in the end portion of the housing, and a remaining part is located in the side portion of the housing; and
third electrodes, disposed in the end portion or the side portion of the housing, wherein
the third electrodes comprise group-C electrodes, and each of the group-C electrodes and an adjacent first electrode may form an electrode pair to generate a microcurrent.

2. The portable beauty instrument according to claim 1, wherein the electrodes comprise two first electrodes, the first electrodes are arc-shaped electrode strips extending along an edge of the housing, and the two first electrodes are respectively located on two opposite sides of the end portion of the housing.

3. The portable beauty instrument according to claim 2, wherein the group-C electrodes comprise two electrodes, the group-C electrodes are disposed in the end portion of the housing, and are located between the two first electrodes, and each of the first electrodes cooperates with an adjacent electrode in the group-C electrodes to form an electrode pair.

4. The portable beauty instrument according to claim 3, wherein the third electrodes further comprise group-B electrodes that can independently work to generate a microcurrent or a radio frequency current, and electrodes in the group-B electrodes are arranged at intervals in a length direction of the first electrode.

5. The portable beauty instrument according to claim 4, wherein the third electrodes are triangularly distributed as a whole, and two electrodes in the group-C electrodes are respectively located on two sides of the group-B electrodes.

6. The portable beauty instrument according to claim 1, wherein the electrodes further comprise:
second electrodes, disposed in the end portion of the housing, wherein the second electrodes cooperate to form an electrode pair that can discharge to the skin.

7. The portable beauty instrument according to claim 6, wherein an outer side surface of the housing has a first region and a second region, an area of the first region is greater than an area of the second region, the second electrodes are disposed in the first region, and the third electrodes are disposed in the second region.

8. The portable beauty instrument according to claim 6, wherein the second electrodes are located at one end of the third electrodes in an extension direction of the first electrode.

9. The portable beauty instrument according to claim 6, wherein the second electrodes comprise a central electrode and several peripheral electrodes, and the several peripheral electrodes are disposed around the central electrode.

10. The portable beauty instrument according to claim 9, wherein the central electrode is a ring-shaped electrode, the peripheral electrode is an arc segment-shaped electrode, and taking the central electrode as a center, the peripheral electrodes are distributed around the circumference of a specified circle at equal intervals.

11. The portable beauty instrument according to claim 6, comprising:
a processing circuit, located in the housing and connected to the electrodes; and
an adapter board, located in the housing and disposed near the end portion, wherein the adapter board has several connection terminals corresponding to the second electrodes and the third electrodes, the end portion has first through-holes corresponding to the second electrodes and the third electrodes, the second electrodes and the third electrodes are electrically connected to the several connection terminals through the first through-holes, and the several connection terminals are connected to the processing circuit.

12. The portable beauty instrument according to claim 11, comprising:
several first fasteners having conductivity;
wherein second through-holes are disposed at positions of the adapter board corresponding to the connection terminals, there are first fastening portions on sides of the second electrode and the third electrode facing the adapter board, and the first fasteners pass through the second through-holes from a side of the adapter board facing away from the second electrodes and the third electrodes, and are fastened to the first fastening portions.

13. The portable beauty instrument according to claim 9, wherein all the peripheral electrodes in the second electrodes are connected through a connection line and/or a connection ring and/or a connection piece.

14. The portable beauty instrument according to claim 1, comprising:
a fastener, wherein
a mounting groove and a through-hole disposed at a groove bottom of the mounting groove are disposed in a connection region between the end portion and the side portion of the housing, there is a fastening portion on a side of the first electrode facing the housing, and the fastener passes through the through-hole from the inside of the housing, and is connected to the fastening portion; and
a sealing ring, sleeved on the fastening portion or the fastener and located between the first electrode and the groove bottom, to seal a gap between the fastening portion and an inner wall of the through-hole or seal a gap between the fastener and the inner wall of the through-hole.

15. The portable beauty instrument according to claim 11, comprising:
a circuit board, disposed between the adapter board and the end portion; and
several light therapy lamps, disposed on a side of the circuit board facing the end portion and connected to the processing circuit through the circuit board, wherein
the end portion has a transparent region corresponding to the light therapy lamp, and the transparent region is configured to shield an external line of sight when the light therapy lamp does not emit light and be capable of transmitting at least a part of light when the light therapy lamp emits light.

16. The portable beauty instrument according to claim 15, comprising:
a mounting bracket, disposed between the adapter board and the end portion and having several positioning holes or positioning slots, wherein there is a light shielding region around the positioning holes or the positioning slots; and
a light guide member, disposed between the mounting bracket and the adapter board, wherein the light guide member comprises a connecting bracket and several light guide columns, and the connecting bracket is connected between the several light guide columns, wherein
the positioning holes or the positioning slots, the light guide columns, and the light therapy lamps are disposed in a one-to-one correspondence.

17. The portable beauty instrument according to claim 1, comprising:
conductive members, connected to at least two of the electrodes and a processing circuit.

18. The portable beauty instrument according to claim 17, further comprising a gating circuit connected to the processing circuit, wherein the electrodes comprise second electrodes, wherein
the conductive members comprise a first conductive member and a second conductive member, the gating circuit is connected between the first conductive member and the second conductive member, and at least two of the second electrodes are connected to the first conductive member; and
at least two of the third electrodes are connected to the second conductive member, and the processing circuit drives the second electrodes, the third electrodes, or the second electrodes and the third electrodes by using the gating circuit.
